# EUROPEAN PATENT SPECIFICATION

(11) **EP 0 556 485 B1**
(45) Date of publication and mention of the grant of the patent: **15.11.1995**
(21) Application number: 92122086.9
(22) Date of filing: 29.12.1992
(51) Int. Cl.: C07K 5/06

(54) **Method for purifying alpha-L-aspartyl-L-phenylalanine methyl ester**
Methode zur Reinigung des Alpha-L-Aspartyl-L-Phenylalanin-Methylesters
Procédé de purification de l'ester-méthylique de l'alpha-L-aspartyl-L-phénylalanine

(30) Priority: 14.01.1992 JP 4890/92
(43) Date of publication of application: 25.08.1993
(73) Proprietor: Ajinomoto Co., Inc., Tokyo 104 (JP)
(72) Inventor: Takemoto, Tadashi, c/o Central Research Lab., Kawasaki-shi, Kanagawa-ken (JP); Sugiyama, Katsumi, c/o Tokai Plant, Yokkaichi-shi, Mie-ken (JP)
(74) Representative: Strehl Schübel-Hopf Groening & Partner

(56) References cited:
- EP-A- 0 256 515
- EP-A- 0 399 605
- EP-A- 0 484 769
- EP-A- 0 514 939
- EP-A- 0 514 940

## Description

This invention relates to a method for purifying α-L-aspartyl-L-phenylalanine methyl ester (hereinafter abbreviated as α-APM) which contains, as impurity, at least 3-benzyl-6-carboxymethyl-2,5-dioxopiperazine (hereinafter abbreviated as DKP), which allows an efficient removal of the impurities.

α-APM is a peptide sweetener whose degree of sweetness is about 200 times as high as that of sucrose. It is a low-calory substance of good quality which has become highly demanded in the recent years. Its stability, however, involves some problems; it is known that it is apt to be cyclized by heat to form DKP. Accordingly, α-APM prepared by any known method unavoidably contains DKP as impurity, and it is a permanent problem to be solved by the producers of the substance to obtain pure α-APM by efficiently removing DKP. Although a variety of methods have been developed in order to solve this problem, any of these methods still involves problems as described in the following. Known is (1) a method utilizing a resin (Japanese Patent Publication No. 35660/1977). The problems involved are decomposition of α-APM during the operation, and complicated process steps for the elution and regeneration of the resin; (2) A method of removing DKP by crystallizing it at a pH of 2 - 2.7 (Japanese Patent Publication No. 40071/1976). This method involves the problem that commonly used equipment, such as an ordinary centrifugal separator cannot be applied since DKP forms very fine needle-like crystals which can hardly be separated continuously on an industrial scale. In order to carry out this process a filter press which has low operational efficiency has to be used. Further known is (3) the n-Butanol extraction method (Japanese Patent Kokai No. 117445/1974) which has the disadvantage that large amounts of she solvent butanol have to be recovered. In addition thereto, any of these known methods requires a crystallization operation after the above described process steps, and therefore at least two steps are necessary in order to purify α-APM by removing DKP.

It is therefore the problem to be solved by the present invention to provide a novel method for purifying α-APM by efficiently removing DKP from the same.

As a result of intensive studies to solve the above-mentioned problems, the present inventors found a method in which α-APM containing DKP as an impurity is brought into contact with hydrochloric acid in an aqueous solvent to deposit a α-APM hydrochloride and DKP and the thus deposited substances are separated from each other by classification, thereby removing DKP into the liquid phase. This invention was accomplished on the basis of these findings.

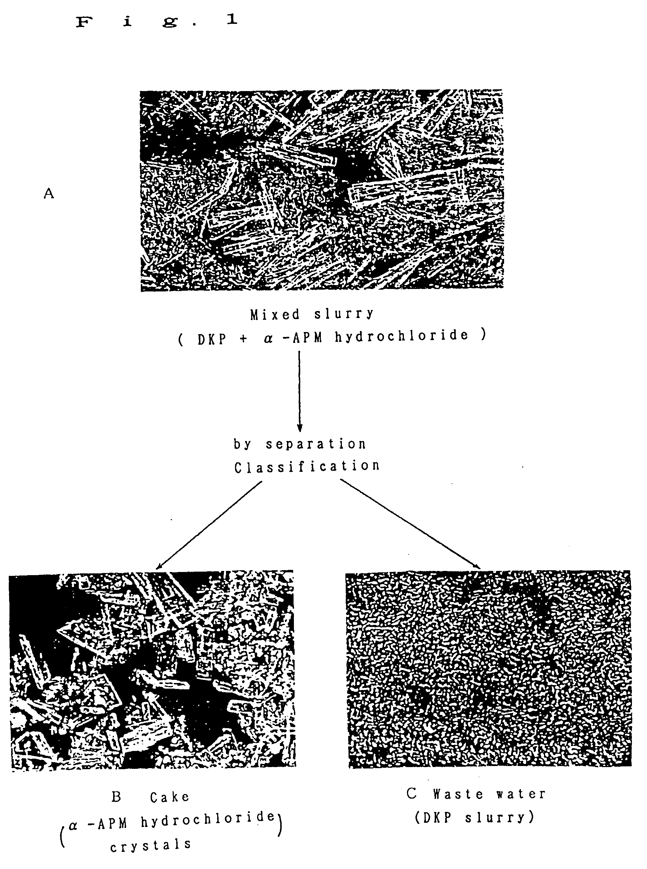

The effectiveness of the method and a preferred embodiment of the apparatus used may by shown with reference to the enclosed drawings. In these drawings figure 1 shows photographs of the starting slurry and the products after the separation by classification.

In more detail, figure 1 A shows the mixed slurry of DKP and α-APM hydrochloride before the separation, figure 1 B shows the obtained cake of α-APM hydrochloride crystals, and figure 1 C shows the DKP slurry removed according to the invention.

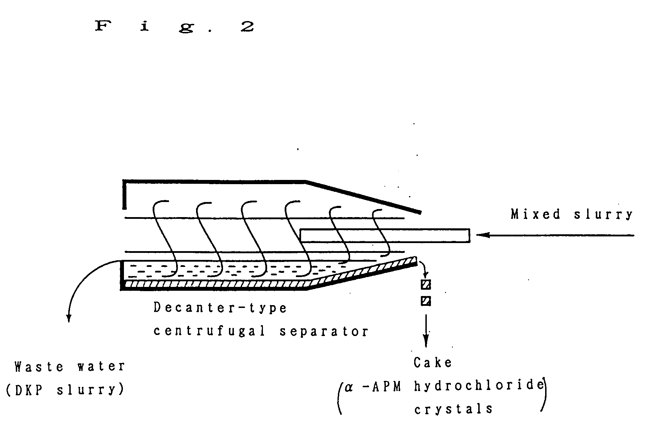

Figure 2 shows a sectional view of the decanter-type centrifugal separator.

The methods of this invention are satisfactorily applicable to the purification of α-APM containing as impurities, in addition to DKP, β-L-aspartyl-L-phenylalanine methyl ester and α-L-aspartyl-L-phenylalanine (impurities commonly observed in the α-APM manufacturing processes) as well as L-aspartic acid, phenylalanine and derivatives thereof (starting materials used in same manufacturing processes), because all of these impurities exist in the liquid phase under the operation conditions in the method of this invention and are therefore efficiently removed into the liquid phase.

The contact of impure α-APM containing DKP with hydrochloric acid may be performed by any method, but is generally performed (1) by dissolving the impure α-APM in an aqueous solvent and adding hydrochloric acid to said solution, (2) by dissolving or suspending the impure α-APM in an aqueous solvent containing hydrochloric acid, or (3) by directly adding hydrochloric acid to the solution of α-APM obtained by the reaction for preparing the same. There is no specific limitation upon the temperature of the contact reaction, but a temperature in the range from 10 to 70°C is generally preferable because an excessively high temperature can accelerate the hydrolysis of α-APM, thus lowering the yield of α-APM hydrochloride. The hydrochloric acid concentration should be 0.5N or higher, but an excessively high concentration accelerates the hydrolysis of α-APM, and hence a concentration in the range from 0.5 to about 4N is generally preferable. In the method (1) or (2) described above, it is also possible that the pH of the α-APM-solution is first adjusted to 2 - 3 by addition of hydrochloric acid to crystallize DKP (by utilizing the property of DKP to be crystallizable at a pH of 3 or less) and then hydrochloric acid is further added to adjust its concentration to a level of 0.5N or higher, thereby crystallizing α-APM hydrochloride. These methods are more preferable because the disadvantageous tendency of DKP crystals to become coagulated can be avoided.

This invention is characterized in that mixed slurry of DKP and α-APM hydrochloride obtained by the method described above is subjected to separation by classification utilizing the difference in particle size between these two kinds of crystals, thereby obtaining the crystals of α-APM as a cake and removing the crystals of DKP into the liquid phase in the form of a slurry. As the methods of separation by classification, are known the sedimentation classification utilizing the gravity sedimentation of particles, the hydraulic classification utilizing pressurized water, the machine classification, and the centrifugal classification utilizing centrifugal force. Of these, the simple, centrifugal classification is the most suitable operation in the methods of this invention. As preferable examples, of the apparatus to be used for the centrifugal classification, may be mentioned a liquid cyclone separator in which the centrifugal force is applied by the rotation of a liquid in a fixed cylinder, and a decanter-type, centrifugal separator using a rotator driven externally.

As is apparent from the foregoing, the method of this invention ensures the efficient purification of α-APM by easily removing DKP and other impurities by simple operations. The following Examples will further illustrate the invention.

### Example 1

A mixture of 46 g α-APM and 9 g of DKP (DKP content in α-APM: 20 weight%) was suspended in 900 ml water, and this suspension was adjusted to pH 4.9 and diluted with water to a total volume of 1000 ml. The resulting suspension was heated at 50°C until a clear solution was obtained, the pH was adjusted to 2.7 by addition of 30 ml of 35%-HCl, and the resulting solution was stirred for one hour to crystallize DKP. After cooling to 10°C, 125 ml of 35%-HCl was further added, and stirring was continued at the same temperature for four hours, giving a mixed slurry of DKP and α-APM hydrochloride (refer to Fig. 1A). This slurry was feeded into a decanter-type, centrifugal separator [Model P-400YJ (rotational speed: 3600 rpm); product of Sharpless Co., Ltd.] at a speed of 20 L/H, thus separating the slurry into a cake of α-APM hydrochloride (refer to Fig. 1B) and DKP slurry (refer to Fig. 1C) (refer to Fig. 2 for this operation). The yield of α-APM hydrochloride cake (*1) was 99%, and the removal rate of DKP crystals into the liquid phase (*2) was 97%.
The amount of DKP contained in the α-APM hydrochloride crystals obtained was 0.2% based on the weight of α-APM.
*1 The yield of α-APM hydrochloride = (α-APM·HCl crystals in cake)/(α-APM·HCl crystals in slurry)
*2 The removal rate of DKP crystals = (DKP crystals in waste water)/(DKP crystals in slurry)

### Example 2

To 100 liters of aqueous solution containing 4.6% α-APM, 0.7% DKP , 3.5% β-L-aspartyl-L-phenylalanine methyl ester, 0.4% L-aspartic acid and 0.2% L-phenylalanine, was added at 50°C, 3 liters of 35%-HCl to adjust the pH to 2.6, and the resulting mixture was stirred for one hour at the same temperature to deposit DKP crystals. This slurry was cooled to 10°C, 13 liters of hydrochloric acid was added, and the mixture was stirred for one hour at the same temperature and then for three hours at 5°C. The mixed slurry of α-APM hydrochloride and DKP thus obtained (HCl concentration: 1.5N) was feeded into a decanter-type centrifugal separator [Model P-660 (rotational speed: 6000 rpm; product of Sharpless Co., Ltd.] at a speed of 600 L/Hr, thus separating into a cake of α-APM hydrochloride and a DKP slurry (waste water). The yield of the α-APM hydrochloride crystals in the case was 100% based on the weight of the same in the feeded slurry, and the removal rate of DKP crystals into the waste water was 93%. The amount of DKP contained in the cake of α-APM hydrochloride crystals was 0.5% based on the weight of α-APM, and no β-L-aspartyl-L-phenylalanine methyl ester, L-aspartic acid or L-phenylalanine was contained in the cake.

## Claims

1. A method for purifying α-L-aspartyl-L-phenylalanine methyl ester containing as impurity at least 3-benzyl-6-carboxymethyl-2,5-dioxopiperazine, characterized in that the impure α-L-aspartyl-L-phenylalanine methyl ester is brought into contact with hydrochloric acid in an aqueous solvent to deposit α-L-aspartyl-L-phenylalanine methyl ester hydrochloride and 3-benzyl-6-carboxymethyl-2,5-dioxopiperazine and the substances thus deposited are separated from each other by classification, thereby removing 3-benzyl-6-carboxymethyl-2,5-dioxopiperazine into the liquid phase.

2. The method according to Claim 1, wherein 3-benzyl-6-carboxymethyl-2,5-dioxopiperazine is first deposited by adjusting the pH to 2 - 3 by addition of hydrochloric acid, and then hydrochloric acid is further added to a concentration of 0.5 - 4N, thereby crystallizing α-L-aspartyl-L-phenylalanine methyl ester hydrochloride.

3. The method according to claim 1 or claim 2, wherein a centrifugal classifier is used to perform the separation by classification.

4. The method according to Claim 3, wherein said centrifugal classifier is a decanter-type centrifugal separator.

5. The method according to any of the claims 1 to 4, wherein the impure α-L-aspartyl-L-phenylalanine methyl ester further contains as impurities β-L-aspartyl-L-phenylalanine methyl ester, α-L-aspartyl-L-phenylalanine, aspartic acid and/or phenylalanine and derivatives thereof and the impurities are removed into the liquid phase.

## Patentansprüche

1. Verfahren zur Reinigung von α-L-Aspartyl-L-phenylalaninmethylester, der als Verunreinigung mindestens 3-Benzyl-6-carboxymethyl-2,5-dioxopiperazin enthält, das dadurch gekennzeichnet ist, daß der verunreinigte α-L-Aspartyl-L-phenylalaninmethylester mit Chlorwasserstoffsäure in einem wäßrigen Losungsmittel in Kontakt gebracht wird, wobei α-L-Aspartyl-L-phenylalaninmethylester-hydrochlorid und 3-Benzyl-6-carboxymethyl-2,5-dioxopiperazin abgeschieden werden, und die so abgeschiedenen Substanzen durch Klassierung voneinander getrennt werden, wobei 3-Benzyl-6-carboxymethyl-2,5-dioxopiperazin in die flüssige Phase überführt wird.

2. Verfahren gemäß Anspruch 1, wobei 3-Benzyl-6-carboxymethyl-2,5-dioxopiperazin zunächst unter Einstellung des pH-Werts auf 2 bis 3 durch Zugabe von Chlorwasserstoffsäure abgeschieden wird und anschließend Chlorwasserstoffsäure bis auf eine Konzentration von 0,5 bis 4 N weiter zugegeben wird, wobei α-L-Aspartyl-L-phenylalaninmethylester-hydrochlorid kristallisiert.

3. Verfahren gemäß Anspruch 1 oder 2, wobei ein Zentrifugalklassierer zur Durchführung der Trennung durch Klassierung verwendet wird.

4. Verfahren gemäß Anspruch 3, wobei der Zentrifugalklassierer ein Zentrifugalabscheider vom Dekantier-Typ ist.

5. Verfahren gemäß einem der Ansprüche 1 bis 4, wobei der ungereinigte α-L-Aspartyl-L-phenylalaninmethylester ferner als Verunreinigungen β-L-Aspartyl-L-phenylalaninmethylester, α-L-Aspartyl-L-phenylalanin, Asparaginsäure und/oder Phenylalanin und Derivate davon enthält und die Verunreinigungen in die flüssige Phase überführt werden.

## Revendications

1. Procédé de purification d'ester méthylique d'α-L-aspartyl-L-phénylalanine contenant comme impureté au moins de la 3-benzyl-6-carboxyméthyl-2,5-dioxopipérazine, caractérisé en ce qu'on met en contact l'ester méthylique d'α-L-aspartyl-L-phénylalanine impur avec de l'acide chlorhydrique dans un solvant aqueux pour déposer du chlorhydrate d'ester méthylique d'α-L-aspartyl-L-phénylalanine et de la 3-benzyl-6-carboxyméthyl-2,5-dioxopipérazine et en ce qu'on sépare les unes des autres les substances ainsi déposées par classement, éliminant de ce fait la 3-benzyl-6-carboxyméthyl-2,5-dioxopipérazine dans la phase liquide.

2. Procédé selon la revendication 1, dans lequel on dépose d'abord la 3-benzyl-6-carboxyméthyl-2,5-dioxopipérazine en ajustant le pH entre 2 et 3 par addition d'acide chlorhydrique et dans lequel on ajoute ensuite encore de l'acide chlorhydrique à une concentration comprise entre 0,5 et 4 N, cristallisant de ce fait le chlorhydrate d'ester méthylique d'α-L-aspartyl-L-phénylalanine.

3. Procédé selon la revendication 1 ou la revendication 2, dans lequel on utilise un classificateur centrifuge pour réaliser la séparation par classement.

4. Procédé selon la revendication 3, dans lequel ledit classificateur centrifuge est un séparateur centrifuge de type appareil à décanter.

5. Procédé selon l'une quelconque des revendications 1 à 4, dans lequel l'ester méthylique d'α-L-aspartyl-L-phénylalanine impur contient de plus, comme impuretés, de l'ester méthylique de β-L-aspartyl-L-phénylalanine, de l'α-L-aspartyl-L-phénylalanine, de l'acide aspartique et/ou de la phénylalanine et leurs dérivés et dans lequel on élimine les impuretés dans la phase liquide.
